# EUROPEAN PATENT SPECIFICATION

(11) **EP 1 284 644 B1**
(45) Date of publication and mention of the grant of the patent: **20.10.2004**
(21) Application number: 01936046.0
(22) Date of filing: 28.05.2001
(51) Int. Cl.: A61B 5/04

(54) **AN ELECTRODE FOR ESTABLISHING ELECTRICAL CONTACT WITH THE SKIN**
ELEKTRODE ZUR HERSTELLUNG EINES ELEKTRISCHEN KONTAKTS MIT DER HAUT
ELECTRODE PERMETTANT D'ETABLIR UN CONTACT ELECTRIQUE AVEC LA PEAU

(30) Priority: 29.05.2000 DK 200000847
(43) Date of publication of application: 26.02.2003
(73) Proprietor: MEDICOTEST A/S, 3650 Olstykke (DK)
(72) Inventor: NIELSEN, Brian, DK-2100 Copenhagen (DK); THOMSEN, Steen, DK-2500 Valby (DK)
(74) Representative: Olsen, Lau Lund
(86) International application number: PCT/DK2001/000369
(87) International publication number: WO 2001/091637

(56) References cited:
- EP-A1- 0 243 053
- US-A- 4 077 397
- US-A- 4 899 754
- US-A- 5 402 780

## Description

### Technical Field

The present invention relates to an electrode for establishing electrical contact with the skin, a method for preparation a ready-for-use electrode, and a use of this ready-for-use electrode. More specifically, this invention relates to physiological electrodes by means of which one or more physiological functions may be monitored or stimulated.

### Technical Background

Electrodes establishing electrical contact with the skin are used for the administration of electrical signals to the body as well as measuring electrical signals generated in or by the body.

Electrical signals may be administered to the body of a patient through skin electrodes for various purposes, including the treatment of fibrillation by administering an electric shock, treatment of pain and promotion of healing. The electric shock counteracts atrial or ventricular fibrillation of the heart and, if the treatment is successful, makes the rhythm of the heart revert to the normal mode.

Electric signals generated in the body may be collected by skin electrodes and monitored on a suitable monitoring device. In particular, the electrical signals of the heart may be monitored on a electrocardiogram (ECG) to monitor the operation of the heart.

Skin electrodes should meet a plurality of requirements to be suitable for supplying or measuring electrical signals, e.g. the skin electrodes must be sufficiently flexible to conform with the patient's body to secure a sufficient contact area, and to display satisfactory adhesion and electrical contact with the patient's body when the electrodes are placed properly.

A special requirement for an electrode is that the electrode suitably should be able to withstand storage without fast deterioration. Prolonged storage is valuable for a variety of reasons. For example, electrodes are frequently a part of the standard emergency equipment used by rescue teams and in remote areas. Therefore, the reliability of the electrodes may be crucial for saving lives. Furthermore, in the absence of a stabile and reliable electrode the emergency equipment should constantly be watched and old not used electrodes must be disposed of.

It is well-known that electrodes may have a tendency to deteriorate during storage resulting in a reduced shelf life. In US 4,895,169 it is speculated that the reason for the deterioration is the presence of saline in the electrically conductive gel. To improve the shelf life it is suggested to use a tin-stannous chloride electrode element. More specifically, it is suggested to use tin as the conductive layer having affixed stannous chloride to the face of the layer pointing towards the electrically conductive gel.

In US 4,674,512 it is suggested to incorporate a stabilizer in the gel to prevent tin salt from reacting with other chemical constituents of the electrically conductive gel. The amount of the stabilizer is preferably sufficient for maintaining the tin ions in solution within the electrically conductive gel. The stabilizer may be an acid group bearing compound such as tartaric acid, a n-alkyl sulfonate, citrate, or sodium nitrate.

US 4,899,754 discloses a method for attachment and detachment of an electrical lead wire to an electrode. The electrical lead wire provides for the electrical communication with the appropriate apparatus. The prior art electrode comprises a release liner arranged between a part of a top insulative layer and an electrically conductive layer or between a part of the electrically conductive layer and an electrically conductive adhesive. Prior to the use of the electrode the release liner is removed and an electrical lead wire is inserted in place of the release liner.

The interface between the electrically conductive gel and the conductive layer is of major importance to the electrically properties of the entire electrode. An effective electrically coupling of the two layers will permit rapid depolarization of the electrode after defibrillation. If the coupling is satisfactory, the electrode will exhibit a relative low and constant DC offset, i.e. one that is constant over time rather than rising over a period of time after having been applied to the skin.

In one aspect, the present invention aims at providing a method for improving the shelf life of electrodes which are suitable for establishing electrical contact with the skin. Especially, it is the purpose of a certain aspect of the invention to provide a method for improving the shelf life of a physiological electrode comprising a electrically conductive gel which during storage is corrosive towards a metal of the electrically conductive layer.

### Disclosure of the Invention

The invention concerns an electrode for establishing electrical contact with the skin, comprising an electrically conductive layer and an electrically conductive gel, the electrically conductive layer being provided with connecting means for establishing electrical communication with a suitable device or instrument, wherein a face of the electrically conductive layer is separated from a face of the electrically conductive gel by an intervening part which is adapted to be removed, characterized in that substantially the entire area of one side of the electrically conductive layer is separated from the electrically conductive gel by means of the intervening part.

It is desired to separate a face of the electrically conductive layer from a face of the electrically conductive gel facing the electrically conductive layer over the entire area of contact. However, in some applications, it may be desired to separate only a part of a face of said electrically conductive layer from at least a part of a face of said electrically conductive gel by the removable intervening part. Sandwiching a removable intervening part between the electrically conductive layer and the electrically conductive gel substantially hinder or impede contact between the two layers. Further, the presence of the removable intervening part separating the electrically conductive layer and the electrically conductive gel impedes or hinders any chemical reaction that otherwise would have taken place in the absence of said layer.

The separation by the intervening part to be removed before use of the electrode may be provided by any suitable means which can be removed so that an intimate contact between the electrically conductive layer and the electrically conductive gel can be obtained. Examples of means for the intervening part may be a sheet of plastic, paper, coated paper, laminate, fabric, or coated fabric. If a sheet of plastic is used for the removable intervening part it can be prepared of polyethylene, polypropylene, polyamide, polyesters, polyethers, polyvinylchloride, or any combination thereof. If a coated paper or fabric is used for the removable intervening part, the coating may be of any suitable slipping agent. Preferably, a siliconized paper is used for the removable intervening part. The paper may be siliconized on one or both sides. Usually, it is sufficient to siliconize the side of the paper in contact with the electrically conductive gel. The silicone provides for a suitable attachment to the electrically conductive gel while being easily removable therefrom. Furthermore, the friction between two layers of siliconized papers is suitable low.

In accordance with one aspect of the present invention the removable intervening part is a single layer. A single layer provides a cost effective separation, because a minimum of material may be used. The single layer is suitable attached to the electrically conductive gel and do not adhere to the electrically conductive layer. Before use of the electrode, the intervening layer is removed by separating the electrically conductive layer from the intervening layer and subsequently pulling in the intervening layer. After removal of the intervening layer, the surface of the electrically conductive layer is contacted with the electrically conductive gel, thereby securing an intimate contact.

In another aspect of the invention, the intervening part comprises two or more stacked layers. For example, the intervening part may comprise 2, 3 or 4 stacked layers. Preferably, the multilayered structure of the removable intervening part is folded, allowing the structure to be prepared from a single sheet.

In one aspect of the invention, the intervening layer exerts forces by removal on at least one of the electrically conductive layer and the electrically conductive gel, forcing said electrically conductive layer and said electrically conductive gel together. Suitably, the intervening part is a double layer prepared from a single sheet which has been folded. The intervening part may be formed such that the layer in contact with the electrically conductive gel is smaller than the layer facing the electrically conductive layer, thereby allowing a part of the layer facing the electrically conductive layer to extent out of the circumference of the other parts of the electrode. When one pulls in this part, the upper part of the intervening layer will slide against the electrically conductive layer and the fold will move in the pulling direction. The pulling force is transformed to a inwardly directed force which will approach the electrically conductive layer and the electrically conductive gel to each other. Preferably, the inward directed force is sufficient for providing an intimate contact between the electrically conductive layer and the electrically conductive gel. Alternatively, or in addition thereto, the intimate contact between the electrically conductive layer and the electrically conductive gel can be secured by pressing with any suitable means, such as the fingers of the operator.

In yet another embodiment, the removable intervening part is a quadruple layer prepared from a single sheet which has been folded. Suitably, a single sheet is folded three times to create a fourfold layer. The part of the sheet comprising the second fold suitably extents outside the circumference of the other parts of the electrode. When one pulls in this part, the first and the third fold is moved in the pulling direction exerting a mechanical force on the electrically conductive layer and the electrically conductive gel, respectively, which forces the electrically conductive layer and the electrically conductive gel together. To obtain a satisfactory coupling between the electrically conductive layer and the electrically conductive gel it may be desired to press these layers together with suitable means, such as the fingers of the operator.

The use of two or more stacked layers allows for a construction of the electrode in which the parts of the electrode comprising the electrically conductive gel and the electrically conductive layer, respectively, can be attached in the area near the circumference on more than 1 side, preferably 2 or 3 sides. In this way, it may be secured that the individual components of the electrode is superimposed, avoiding a mismatch of the electrode parts and securing an electrode which will operate properly after bringing the electrically conductive layer in contact with the electrically conductive gel.

During storage the part of the electrode comprising the electrically conductive gel and the part comprising the electrically conductive layer need not to be superimposed. In an embodiment also intended to be comprehended by the scope of protection conferred by the claims, the electrically conductive gel is provided on one part of a film or laminate and the electrically conductive layer is provided on another part. Furthermore, a removable sheet is provided such that at least the electrically conductive gel is covered. However, preferably also the electrically conductive layer is covered by the removable sheet. Before application of the electrode the removable sheet or laminate is removed and the parts of the sheet comprising the electrically conductive gel and the electrically conductive layer, respectively, are attached to each other, preferably by bending the sheet to superimpose the electrically conductive gel and the electrically conductive layer. A preferred method of removing the intervening part is to bend the assembly such that the electrically conductive gel and the electrically conductive layer, respectively, are superimposed and subsequently remove the intervening part as previously described for a double layered intervening part, i.e. to pull in a part of the intervening part in intimate contact with the electrically conductive layer and extending outside the circumference of the other parts of the electrode.

The electrically conductive layer may be formed of any suitable means which can conduct the electric current satisfactory. For example, the electrically conductive layer may be a metallic layer comprising tin, aluminium, zinc, lead, or any alloy comprising said metals. The metallic layer may be covered on the side facing the intervening part with an agent which improves one or more of the electrical characteristics. Suitably, the metallic layer may be covered with a salt of a metal contained in the metallic layer. As an example, an electrically conductive layer comprising tin may be covered with stannous chloride to improve the conductivity when contacted with the electrically conductive gel.

Alternatively, the electrically conductive layer may be a conductive ink printed on a suitable backing. In a preferred embodiment the ink comprises a suitable binding agent and particles of a metal and a salt of said metal. The metal may, for instance, be tin, aluminium, zinc, lead, or silver. Silver is preferred due to the good conductive properties thereof. The anion of the particles of the metal salt may for example be chloride, bromide, or iodide. In one aspect of the invention, the electrically conductive layer comprises Ag and AgCl particles distributed in the binding agent.

Various electrically conductive gels well-known to the person skilled in the art may be used in the electrode according to the invention. Preferred gels are prepared of hydrophilic polymers. In the following, gels prepared of hydrophilic polymers will be termed hydrogels. Hydrogels comprise an amount of water, which increases the skin compatibility and lower the electrical resistance.

The hydrophilic polymer may for instance be selected from the group consisting of polyacrylate, polymethacrylate, polyacrylamide, poly(vinyl alcohol), poly(ethylene oxide), poly(ethylene imine), carboxymethylcellulose, methyl cellulose, poly(acryl amide sulphonic acid), polyacrylonitril, poly(vinylpyrrolidone), agar, dextran, dextrin, carrageenan, xanthan, and guar.

The electrically conductive gel is preferably a flexible stiff gel which maintains the integrity during storage and applications. However, the electrically conductive gel may be in the form of a viscous paste or creme, if so desired.

The pH of the electrically conductive gel may have any suitable value, i.e. the gel may be acidic, neutral or alkaline. In one aspect of the present invention, the electrically conductive gel provides for an acidic or an alkaline corrosion of the electrically conductive layer. The acid or alkaline electrically conductive gel, respectively, may be provided in any suitable way. In one embodiment a mineral or organic acid or base, that provides for the eventually obtained pH, is added to the gel during the preparation thereof. Examples of mineral or organic acids that may be used are hydrochloric acid, sulphuric acid, nitric acid, phosphorus acid, acetic acid, formic acid, benzoic acid, and sulfonic acid. Examples of mineral or organic alkaline substances that may be used are ammonia, potassium hydroxide, sodium hydroxide, calcium hydroxide, pyridine, and aniline. In another embodiment, which is explained in more detail below, the polymers of the hydrogel structure itself contains acid or alkaline groups. In a third embodiment of the invention, a combination of the two preceding embodiments are used, i.e. the gel contains a mineral or organic acid or base added during the preparation as well as polymers carrying acid or alkaline groups.

If the corrosion of the metallic layer is performed in an acidic environment, it is preferred that the polymer comprises acid groups, such as carboxylic, sulphonic or nitric groups. In acidic environments such groups will predominantly be anionic and may thus be capable of transferring a cation carrying a charge between the skin of the patient and the metallic layer. A preferred polymer is polyacrylate or polymethacrylate, or a co-polymer containing acrylic acid or methacrylic acid as one of its monomers.

A polyacrylate at low pH may contain a fairly large amount of water providing a sticky gel with an ability to penetrate the small pores of the skin. In a preferred embodiment of the invention the content of water in the hydrogel is above 50 % by weight, more preferred above 70 % by weight, if the pH of the gel is between 1 and 3. The satisfactory coupling between the gel and the surface of the skin of a patient results in a low skin impedance. The satisfactory coupling also ensures a high energy transfer resulting in substantially no burning of the skin. Furthermore, the electrode adheres well to the skin of the patient and remains in position during operation even if tension is applied thereto.

If the corrosion of the metallic layer is provided in an alkaline environment, it is preferred that the ionizable groups of the polymeric structure are basic groups, such as amine, imine, or amide groups. In alkaline environments, such groups will predominantly be cationic and may thus be capable of carrying a free anion in the gel.

Preferably, the gel provides an acidic corrosion of the metallic layer. The pH of the electrically conductive gel may be chosen in dependence of the selected metallic layer and may be determined by the person skilled in the art through routine experiments.

The pH of the electrically conductive gel is preferably between 0 and 4, more preferred between 1 and 3. The pH is selected as a trade-off between skin compatibility and sufficient corrosion of the metallic layer. Therefore, the preferred metals for the electrically conductive metallic layer is selected among metals having a high sensitivity to acid or base. Preferred metals include tin, aluminium, zinc, and lead and any combination thereof. Tin is the most preferred metal for the metallic layer. The purity of the used metal is usually high. Preferably, the purity is 99 % by weight or more. The thickness of the metallic layer is not of particular importance to the present invention. A thickness of 0.05 mm has proved to be useful.

In order to be electrically conductive the hydrogel contains electrolytes, which carries the electrical charges. However, the presence of electrolytes also increase the tendency of the electrically conductive gel to be aggressive toward the electrically conductive layer even though the pH of the hydrogel is close to neutral pH area. This tendency is attenuated if the gel is acidic or alkaline.

Even though some ions may be etched from the metallic layer and may serve to transfer an electrical charge from the metallic layer to the skin surface, it may be desired to add further ions to the gel to improve the conductivity. The ions may be added as an ionizable salt. In principle, any ions having the ability to move in the gel may be used. However, preferred ionizable salts are KCl, KBr, NaCl, AgCl or SnCl2.

In one aspect of the present invention it pertains to an electrode in which a electrically conductive gel is used, wherein the pH of the gel is chosen so as to provide for a corrosion of the electrically conductive layer. Without intending to limit the scope of the invention to a specific explanation or theory, it is at present believed that the chemical attack of the metallic layer provides a diminished impedance at the interface between the metallic layer and the acidic gel. The chemical attack will result in the creation of pits in the surface of the metallic layer, thus increasing the surface area so that the electrical contact between the gel and the metallic layer is improved. It is also believed that the generation of a relatively high concentration of metallic ions at the interface contributes to the availability of current carriers when a current is applied, resulting in a reduced tendency to build-up charge, i.e. to serve as a capacitor.

Besides the electrically conductive layer and the electrically conductive gel the electrode suitably comprises further parts. Preferably, the face of the metallic layer opposite to the face attached to the electrically conductive gel has a suitable insulating cover in order to reduce the risk that the operator will get an electric shock or interact with the measurements. The insulating cover is preferably prepared of a polymeric material such as polyolefine, e.g. polyethylene or polypropylene.

The electrically conductive layer may be connected to instruments or devices that are utilized in connection with skin electrodes in any suitable way. The connecting means includes the direct physical attachment of an electrically conductive wire to the electrically conductive layer by soldering, crocodile clip, rivetting or the like, as well as suitable entities adapted to mate a corresponding part of a connector wire. In one aspect of the invention the electrically conductive layer is provided with a first end of a wire suitable for conducting elect:rical current, the end being fastened to the electrically conductive layer by suitable means such as soldering or rivetting. The other end of the wire may be provided with an appropriate plug for establishing electrical connection with the instrument or device, either directly or via a suitable connecting wire. In another aspect of the invention, it is preferred that the electrically conductive layer is connected with a point adapted to mate with a corresponding portion of a connector, because, especially in an emergency situation, the electrodes may easily be connected to the devices or the instrument. Furthermore, the presence of a point adapted to mate with the corresponding parts of a connector of an instrument or device allows the electrode to be a disposable article. Thus, the electrode may be adapted for single use and may be disposed of in any suitable way.

Whereas the electrically conductive gel used in the present invention is preferably a hydrogel having the ability to adhere to the skin of the patient, it may be preferred to cover the face opposing the face in contact with the electrically conductive layer by a second or further electrically conductive skin adhering layer(s) having a pH more compatible with the skin of the patient. The pH of the second gel layer is preferably 5-9.

During storage the surface of the electrically conductive intended to adhere to the skin is suitably provided with a liner. Immediately before use, the liner is removed and the electrode is attached to the skin of a subject.

The electrode according to the invention may be stored in any suitable container. Preferably, the container is formed of a material which substantially impede or prevent permeation of gases. In a preferred embodiment, the electrode is packed in a bag, the walls of which may be composed of any material or combination of materials which can impede to a substantially extent the permeation of gases from the inside of the bag to the outside as well as gases in the surrounding air to the inside of the bag.

A single material for the walls of the bag is generally not able to fulfil all the functions desired for the bag. Therefore, a laminate of several layers of material is generally used. Due to the properties of aluminium to impede permeation of oxygen, it is preferred to include a layer of aluminium or aluminium alloy in the laminate. Besides the layer of aluminium or aluminium alloy the laminate suitably comprises one or more films of a plastic material. The plastic material may be selected from the group consisting of low-density polyethylene, high-density polyethylene, polypropylene, and polyamide. Preferably, the layer of aluminium is provided with at least one layer of a plastic material on each side to avoid damage of the metallic layer.

The bag is suitably prepared by superimposing two sheets and welding the edges with heat supply. In a preferred embodiment of the invention an edge of the intervening part is fixed to an edge of the bag in which the electrode is stored, allowing for the removal of the intervening part when the electrode is pulled out of the bag. Suitably, the edge of the intervening part is sandwiched between edges of the two sheets and fixed by welding in the part of the bag opposite to the direction of pulling, thereby allowing for an easy and convenient removal of the intervening layer before use of the electrode. It may be preferred to provide the bag with suitable means for securing the contact between the electrically conductive layer and the electrically conductive gel during or after the removal of the intervening part. Examples of such means are a pair of rolls through which the two parts of the electrode passes when pulled out of the bag.

In an aspect of the present invention it relates to a method for production of a ready-for-use electrode for establishing electrical contact with the skin as defined in claim 15.

The electrode provided may be any electrode as described in the preceding part of the description. In one aspect, the electrode is formed of an electrically conductive layer and an electrically conductive gel reacting with each other upon contact.

The electrode is usually connected to an appropriate device or instrument through suitable connecting means. While the connection means may be any entity which establishes an electrical contact between the electrode and the device or instrument, it is, in one aspect of the invention, preferred that the connection means is a point adapted to mate with a corresponding portion of a connector.

In another aspect of the invention, the electrically conductive layer is provided with a first end of a wire suitable for conducting electrical current and the other end of the wire in adapted to establish electrical connection with an instrument or a device, either directly or via a suitable connecting wire. Preferably, the end of the wire in fastened to the electrically conductive layer by a soldering or a rivet.

Suitably, the removable intervening part comprises two or more bended layers. Preferably, the removal of the intervening part in step (ii) exerts forces on at least one of said electrically conductive layer and said electrically conductive gel, forcing said electrically conductive layer and said electrically conductive gel together.

The electrode according to the present invention may be used for a variety of applications, including monitoring, stimulation, therapeutical and surgical applications.

The monitoring applications include any measurement of the condition of the muscles or nerves of the human or animal body. Specific examples for the use of the electrode according to the present invention for monitoring applications are ECG, EMG (electromyography) and EEG (electroencephalography).

The stimulation applications include any method for stimulation of the muscles or nerves of the human or animal body. Specific examples of the use of the electrode according to the present invention for stimulating applications are for defibrillation, pacing and pain relief.

Examples of therapeutical applications of the electrode according to the present invention are for electro therapy of muscles and nerves.

The electrode according to the present invention may also be used for surgical applications as grounding plate. A grounding plate is used in a special surgical technique wherein the tissue of the patient is cut with a needle supplied with a high voltage. When the needle supplied with the high voltage comes into contact with the skin heat will be developed and the tissue may be cut. The grounding plate is used to close the electrical circuit. To avoid burning the grounding plate usually has a fairly large contact area with the skin.

### Short Description of the Figures

Fig. 1 shows an exploded view of an embodiment of the invention having a double layer prepared from a single sheet which has been folded.
Fig. 2 shows an exploded view of another embodiment according to the present invention having a quadruple layer prepared from a single sheet which has been folded three times.

### Detailed Description of the Figures

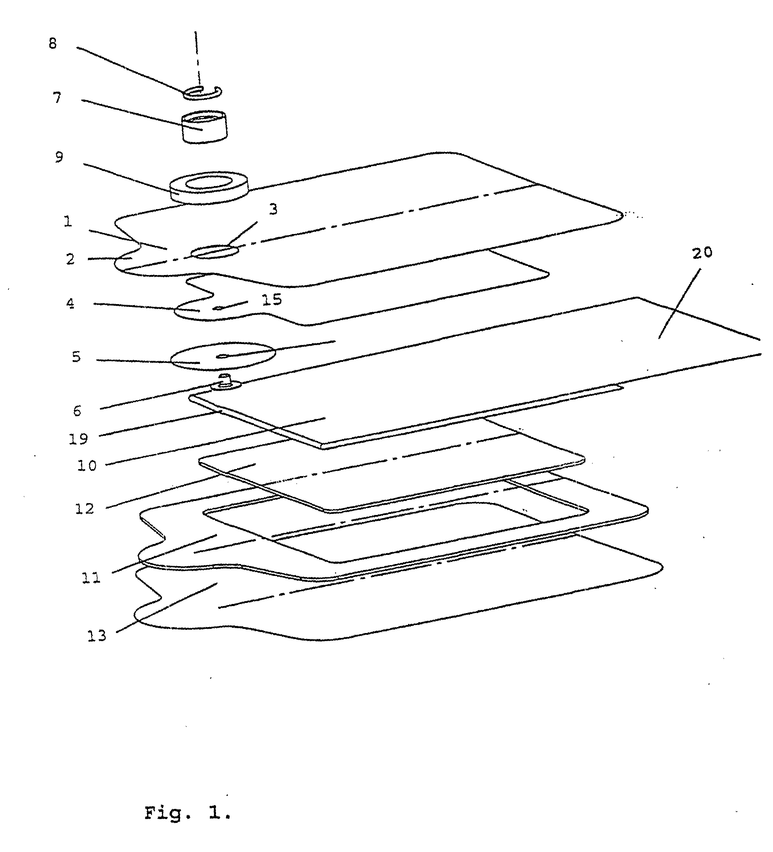

Fig. 1 shows an exploded view of an electrode according to a preferred embodiment of the invention. The embodiment shown in Fig. 1 includes an electrically insulating backing or substrate 1. The substrate 1 has an oblong overall shape and an ear 2 attached to the circumference thereof. In addition, the substrate 1 comprises an aperture 3 near the ear 2. An electrically conductive layer 4 of tin metal foil is centred attached to the substrate 1. On the face of the electrically conductive layer opposite the substrate, a relief film 5 is placed in the area in the vicinity of the point. A small hole 14 is provided in the relief film and a hole 15 is also provided in the electrically conductive layer 4. Through the holes 14 and 15 a rivet 6 is provided. The rivet 6 fastens a cup 7 to the electrically conductive layer 4 to provide an effective electrical contact. Inside the cup, a snap ring 8 is placed to allow for easy and fast connection. The cup 7 protrudes through the aperture 3. Around the cup, an insulating annular spacer 9 of a foam material is attached.

The area delimited by a frame 11 and the release liner 13 is filled with an electrically conductive gel 12. Between the electrically conductive layer 4 and the electrically conductive gel 12 a removable intervening part 10 is provided to avoid contact between these. The removable intervening part 10 is a double layer prepared of a sheet which has been folded. The double layer has a fold 19 in the direction of the connector and a pulling part 20.

Prior to the use of the skin electrode according to the embodiment shown in Fig. 1, the intervening layer and the release liner is removed. Subsequently, the surface of the gel is brought into contact with the skin of a patient. The skin adhering ability of the gel ensures that the electrode remains in position.

The intervening layer 10 is removed by pulling in the pulling part and at the same time holding in the ear 2. The upper layer of the double layer is slid, when exerted to pulling, against the electrically conductive layer 4 and the fold 19 is moved in the direction of pulling. The lower part of the double layer is adhered to the electrically conductive gel 12 and the fold is, when moved in the direction of pulling, producing an inwardly directed force. The inwardly directed force is supplemented by manually pressing the parts of the electrode comprising the electrically conductive layer and the electrically conductive gel, respectively, together with the fingers, thus ensuring an intimate contact. After removal of the removable intervening layer 10, the release liner 13 is removed before applying the electrode to the skin.

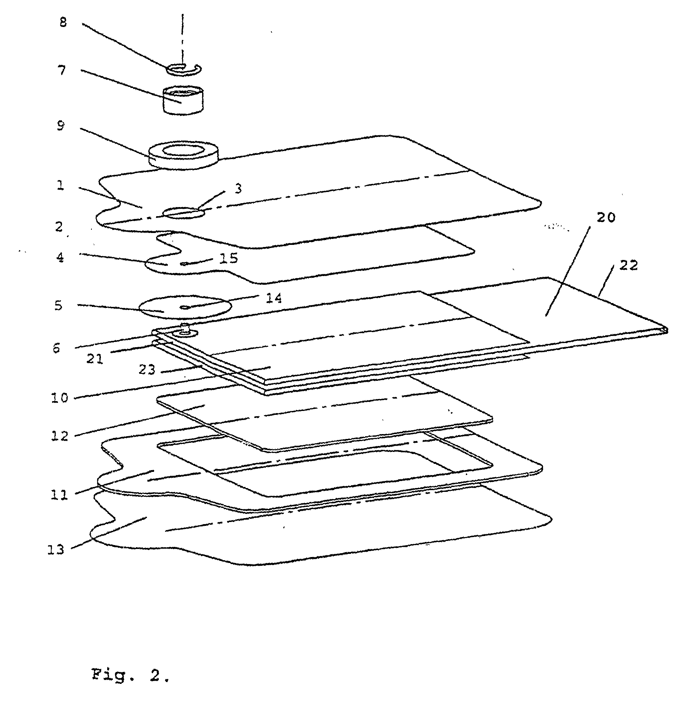

Fig. 2 shows an exploded view of another embodiment of the present invention, wherein a three times folded quadruple layer is used as the removable intervening part.

The composition of the electrode is, apart from the intervening layer, identical to the electrode shown on Fig. 1. The quadruple layer is prepared from a single sheet folded three times. Thus, the sheet contains three folds 21, 22, and 23. The first and the third fold, 21 and 23, is placed in the direction of the connector and the second fold 22 is in the opposite direction. The part of the intervening layer comprising the second bulge 22 is extended outside the circumference of the other part of the electrode.

When pulling in the intervening part comprising the second bulge 22 and simultaneously holding in the ear of the electrode, the first and the third fold (21, 23) are moved in the direction of the pulling, i.e. away from the connector. The upper part of the intervening layer is removable attached to the electrically conductive layer 4 and the lower part is removable attached to the electrically conductive gel 12. The movement of the folds create an inwardly directed mechanical force which will force the electrically conductive gel and the electrically conductive layer together.

Optionally, the two parts of the electrode comprising the electrically conductive layer and the electrically conductive gel, respectively, may be pressed together, either manually with the fingers or by a pair of rolls to ensure a proper physical contact between these two parts.

## Claims

1. An electrode for establishing electrical contact with the skin, comprising an electrically conductive layer (4) and an electrically conductive gel (12), the electrically conductive layer (4) being provided with connecting means (6, 7, 8, 9) for establishing electrical communication with a suitable device or instrument, wherein a face of the electrically conductive layer (4) is separated from a face of the electrically conductive gel (12) by an intervening part (10) which is adapted to be removed, **characterized in that** substantially the entire area of one side of the electrically conductive layer (4) is separated from the electrically conductive gel (12) by means of the intervening part (10).

2. An electrode according to claim 1, wherein the intervening part (10) is a single removable layer.

3. An electrode according to claim 1 or 2, wherein the intervening part (10) exerts forces upon removal on at least one of said electrically conductive layer (4) and said electrically conductive gel (12), forcing said electrically conductive layer (4) and said electrically conductive gel (12) together.

4. An electrode according to any one of the preceding claims, wherein the removable intervening part (10) comprises two or more stacked layers.

5. An electrode according to claim 4, wherein the intervening part (10) is a double layer prepared from a single sheet which has been folded.

6. An electrode according to claim 4, wherein the intervening part (10) is a quadruple layer prepared from a single sheet which has been folded three times.

7. An electrode according to any one of the preceding claims, wherein said intervening part (10) comprises a siliconized layer.

8. An electrode according to claim 1, wherein the electrically conductive layer (4) comprises a binding agent as well as a metal and a salt of said metal.

9. An electrode according to claim 8, wherein the electrically conductive layer (4) comprises Ag and AgCl particles distributed in the binding agent.

10. An electrode according to any one of claims 1 to 6, wherein the electrically conductive layer (4) comprises tin, aluminium, zinc, silver, or lead.

11. An electrode according to any one of the preceding claims, wherein the electrically conductive layer (4) is provided with a first end of a wire suitable for conducting electrical current and the other end of the wire is adapted to establish electrical connection with an instrument or a device, either directly or via a suitable connecting wire.

12. An electrode according to claim 11, wherein the end of the wire is fastened to the electrically conductive layer by a soldering or a rivet.

13. An electrode according to any one of the preceding claims, wherein the electrically conductive gel (12) is acid or alkaline so as to provide corrosion of the metal of the electrically conductive layer (4).

14. An electrode according to any one of the preceding claims and a bag in which the electrode is stored, wherein an edge of the intervening part (10) is fixed to an edge of the bag in which the electrode is stored, allowing for the removal of the intervening part (10) when the electrode is pulled out of the bag.

15. A method for preparation of a ready-for-use electrode for establishing electrical contact with the skin, said method comprising providing an electrode which comprises an electrically conductive layer (4) and an electrically conductive gel (12), wherein a face of the electrically conductive layer (4) is separated from a face of the electrically conductive gel (12) by an intervening part (10),
removing the intervening part (10), and
securing an intimate contact between said face of the electrically conductive layer (4) and said face of the electrically conductive gel (12) after removal of said intervening part (10), **characterized by**
substantially the entire area of one side of the electrically conductive layer (4) being separated from the electrically conductive gel (12) by means of the intervening part (10) before removal of the intervening part (10).

16. A method according to claim 15, wherein the electrically conductive layer (4) and the electrically conductive gel (12) react on contact.

17. A method according to claim 15 or 16, wherein the electrically conductive layer (4) is provided with connecting means (6, 7, 8, 9) for establishing electrical communication with a suitable device or instrument.

18. A method according to any one of claims 15 to 17, wherein the electrically conductive layer (4) is provided with a first end of a wire suitable for conducting electrical current and the other end of the wire is adapted to establish electrical connection with an instrument or a device, either directly or via a suitable connecting wire.

19. A method according to claim 18, wherein the end of the wire is fastened to the electrically conductive layer (4) by a soldering or a rivet.

20. A method according to any one of claims 15 to 19, wherein the removable intervening part (10) comprises two or more stacked layers.

21. A method according to any one of claims 15 to 20, wherein the intervening layer (10) by removal exerts forces on at least one of said electrically conductive layer (4) and said electrically conductive gel (12), forcing said electrically conductive part (4) and said electrically conductive gel (12) together.

## Patentansprüche

1. Elektrode zur Herstellung eines elektrischen Kontakts mit der Haut, umfassend eine elektrisch leitende bzw. leitfähige Schicht (4) und ein elektrisch leitendes bzw. leitfähiges Gel (12), wobei die elektrisch leitfähige Schicht (4) mit Verbindungsmitteln (6, 7, 8, 9) zum Ausbilden bzw. Herstellen einer elektrischen Wechselwirkung bzw. Verbindung mit einer geeigneten Vorrichtung oder einem Instrument versehen ist, wobei eine Seite bzw. Fläche der elektrisch leitfähigen Schicht (4) von einer Seite des elektrisch leitfähigen Gels (12) durch ein zwischenliegendes Teil (10) getrennt ist, welches adaptiert ist, um entfernt zu werden, **dadurch gekennzeichnet, daß** im wesentlichen der gesamte Bereich bzw. die ganze Fläche einer Seite der elektrisch leitfähigen Schicht (4) von dem elektrisch leitfähigen Gel (12) mittels des zwischenliegenden Teils (10) getrennt ist.

2. Elektrode nach Anspruch 1, wobei das zwischenliegende Teil (10) eine einzige entfernbare Schicht ist.

3. Elektrode nach Anspruch 1 oder 2, wobei das zwischenliegende Teil (10) Kräfte beim Entfernen auf wenigstens eines aus der elektrisch leitfähigen Schicht (4) und dem elektrisch leitfähigen Gel (12) ausübt, welche die elektrische leitfähige Schicht (4) und das elektrisch leitfähige Gel (12) zueinander zwingen bzw. beaufschlagen.

4. Elektrode nach einem der vorhergehenden Ansprüche, wobei das entfernbare zwischenliegende Teil (10) zwei oder mehr gestapelte Schichten umfaßt.

5. Elektrode nach Anspruch 4, wobei das zwischenliegende Teil (10) eine Doppelschicht ist, die aus einem einzigen Blatt hergestellt ist, welches gefaltet wurde.

6. Elektrode nach Anspruch 4, wobei das zwischenliegende Teil (10) eine Vierfachschicht ist, die aus einem einzigen Blatt gebildet ist, welches dreimal gefaltet wurde.

7. Elektrode nach einem der vorhergehenden Ansprüche, wobei das zwischenliegende Teil (10) eine siliconisierte Schicht umfaßt.

8. Elektrode nach Anspruch 1, wobei die elektrisch leitfähige Schicht (4) ein Bindeagens bzw. -mittel ebenso wie ein Metall und ein Salz des Metalls umfaßt.

9. Elektrode nach Anspruch 8, wobei die elektrisch leitfähige Schicht (4) Agund AgCl-Teilchen umfaßt, die in dem Bindeagens verteilt sind.

10. Elektrode nach einem der Ansprüche 1 bis 6, wobei die elektrisch leitfähige Schicht (4) Zinn, Aluminium, Zink, Silber oder Blei umfaßt.

11. Elektrode nach einem der vorhergehenden Ansprüche, wobei die elektrisch leitfähige Schicht (4) mit einem ersten Ende eines Drahts versehen ist, der geeignet ist, um elektrischen Strom zu leiten, und das andere Ende des Drahts adaptiert ist, um eine elektrische Verbindung mit einem Instrument oder einer Vorrichtung entweder direkt oder über einen geeigneten Verbindungsdraht auszubilden.

12. Elektrode nach Anspruch 11, wobei das Ende des Drahts an die elektrisch leitfähige Schicht durch Löten oder eine Niete festgelegt ist.

13. Elektrode nach einem der vorhergehenden Ansprüche, wobei das elektrisch leitfähige Gel (12) sauer oder alkalisch ist, um eine Korrosion des Metalls der elektrisch leitfähigen Schicht (4) zu bewirken.

14. Elektrode nach einem der vorhergehenden Ansprüche und eine Tasche bzw. ein Beutel, in welcher(m) die Elektrode gespeichert ist, wobei eine Kante bzw. ein Rand des zwischenliegenden Teils (10) an eine Kante des Beutels festgelegt ist, in welchem die Elektrode gespeichert ist, wobei dies die Entfernung des zwischenliegenden Teils (10) ermöglicht, wenn die Elektrode aus dem Beutel gezogen ist.

15. Verfahren zur Herstellung einer zur Verwendung fertigen bzw. einsatzbereiten Elektrode zum Ausbilden bzw. Herstellen eines elektrischen Kontakts mit der Haut, wobei das Verfahren umfaßt
ein Bereitstellen einer Elektrode, welche eine elektrisch leitende bzw. leitfähige Schicht (4) und ein elektrisch leitendes bzw. leitfähiges Gel (12) umfaßt, wobei eine Seite bzw. Fläche der elektrisch leitfähigen Schicht (4) von einer Seite des elektrisch leitfähigen Gels (12) durch ein zwischenliegendes Teil (10) getrennt wird,
ein Entfernen des zwischenliegenden Teils (10), und
ein Sichern eines innigen Kontakts zwischen der Seite der elektrisch leitfähigen Schicht (4) und der Seite des elektrisch leitfähigen Gels (12) nach einer Entfernung des zwischenliegenden Teils (10),
**dadurch gekennzeichnet, daß**
im wesentlichen die gesamte Fläche von einer Seite der elektrisch leitfähigen Schicht (4) von dem elektrisch leitfähigen Gel (12) mittels des zwischenliegenden Teils (10) vor einem Entfernen des zwischenliegenden Teils (10) getrennt wird.

16. Verfahren nach Anspruch 15, wobei die elektrisch leitfähige Schicht (4) und das elektrisch leitfähige Gel (12) bei bzw. auf Kontakt reagieren.

17. Verfahren nach Anspruch 15 oder 16, wobei die elektrisch leitfähige Schicht (4) mit Verbindungsmitteln (6, 7, 8, 9) zum Ausbilden bzw. Aufbauen einer elektrischen Wechselwirkung bzw. Verbindung mit einem geeigneten Gerät oder Instrument versehen wird.

18. Verfahren nach einem der Ansprüche 15 bis 17, wobei die elektrisch leitfähige Schicht (4) mit einem ersten Ende eines Drahts versehen wird, welcher fähig ist, elektrischen Strom zu leiten, und das andere Ende des Drahts adaptiert wird, um eine elektrische Verbindung mit einem Instrument oder einer Vorrichtung entweder direkt oder über einen geeigneten Verbindungsdraht auszubilden.

19. Verfahren nach Anspruch 18, wobei das Ende des Drahts an die elektrisch leitfähige Schicht (4) durch Löten oder einen Niet festgelegt wird.

20. Verfahren nach einem der Ansprüche 15 bis 19, wobei das entfembare zwischenliegende Teil (10) zwei oder mehr gestapelte Schichten umfaßt.

21. Verfahren nach einem der Ansprüche 15 bis 20, wobei die zwischenliegende Schicht (10) durch Entfernen Kräfte auf wenigstens eine aus der elektrisch leitfähigen Schicht (4) und dem elektrisch leitfähigen Gel (12) ausbildet bzw. ausübt, welche das elektrisch leitfähige Teil (4) und das elektrisch leitfähige Gel (12) zueinander zwingen bzw. beaufschlagen.

## Revendications

1. Électrode permettant d'établir un contact électrique avec la peau, comprenant une couche électroconductrice (4) et un gel électroconducteur (12), la couche électroconductrice (4) étant dotée de moyens de connexion (6, 7, 8, 9) pour établir une communication électrique avec un dispositif ou un instrument approprié, dans laquelle une face de la couche électroconductrice (4) est séparée d'une face du gel électroconducteur (12) par une partie intermédiaire (10) qui est adaptée pour être amovible, **caractérisée en ce que** sensiblement la totalité de la surface d'un côté de la couche électroconductrice (4) est séparée du gel électroconducteur (12) au moyen de la partie intermédiaire (10).

2. Électrode selon la revendication 1, dans laquelle la partie intermédiaire (10) est une couche amovible unique.

3. Électrode selon la revendication 1 ou 2, dans laquelle la partie intermédiaire (10) exerce des forces lorsqu'elle est enlevée sur au moins l'un de ladite couche électroconductrice (4) et dudit gel électroconducteur (12), forçant ladite couche électroconductrice (4) et ledit gel électroconducteur (12) ensemble.

4. Électrode selon l'une quelconque des revendications précédentes, dans laquelle la partie intermédiaire amovible (10) comprend deux couches empilées ou plus.

5. Électrode selon la revendication 4, dans laquelle la partie intermédiaire (10) est une couche double préparée à partir d'une feuille unique qui a été pliée.

6. Électrode selon la revendication 4, dans laquelle la partie intermédiaire (10) est une couche quadruple préparée à partir d'une feuille unique qui a été pliée trois fois.

7. Électrode selon l'une quelconque des revendications précédentes, dans laquelle ladite partie intermédiaire (10) comprend une couche siliconée.

8. Électrode selon la revendication 1, dans laquelle la couche électroconductrice (4) comprend un agent de liaison, ainsi qu'un métal et un sel dudit métal.

9. Électrode selon la revendication 8, dans laquelle la couche électroconductrice (4) comprend des particules d'Ag et d'AgCl distribuées dans l'agent de liaison.

10. Électrode selon l'une quelconque des revendications 1 à 6, dans laquelle la couche électroconductrice (4) comprend de l'étain, de l'aluminium, du zinc, de l'argent, ou du plomb.

11. Électrode selon l'une quelconque des revendications précédentes, dans laquelle la couche électroconductrice (4) est dotée d'une première extrémité d'un fil approprié pour conduire le courant électrique et l'autre extrémité du fil est appropriée pour établir une connexion électrique avec un instrument ou un dispositif, directement ou via un fil de connexion approprié.

12. Électrode selon la revendication 11, dans laquelle l'extrémité du fil est fixée à la couche électroconductrice par une soudure ou un rivet.

13. Électrode selon l'une quelconque des revendications précédentes, dans laquelle le gel électroconducteur (12) est acide ou alcalin de manière à causer la corrosion du métal de la couche électroconductrice (4).

14. Électrode selon l'une quelconque des revendications précédentes et sac dans lequel l'électrode est stockée, dans lesquels un bord de la partie intermédiaire (10) est fixé sur un bord du sac dans lequel l'électrode est rangée, permettant d'enlever la partie intermédiaire (10) lorsque l'électrode est sortie du sac.

15. Procédé pour préparer une électrode prête à l'emploi pour établir un contact électrique avec la peau, ledit procédé comprenant :
fournir une électrode qui comprend une couche électroconductrice (4) et un gel électroconducteur (12), dans laquelle une face de la couche électroconductrice (4) est séparée d'une face du gel électroconducteur (12) par une partie intermédiaire (10),
enlever la partie intermédiaire (10), et
assurer un contact intime entre ladite face de la couche électroconductrice (4) et ladite face du gel électroconducteur (12) après avoir enlevé ladite partie intermédiaire (10),
**caractérisé en ce que** sensiblement la totalité de la surface d'un côté de la couche électroconductrice (4) est séparée du gel électroconducteur (12) au moyen de la partie intermédiaire (10) avant que la partie intermédiaire (10) ne soit enlevée.

16. Procédé selon la revendication 15, dans lequel la couche électroconductrice (4) et le gel électroconducteur (12) réagissent au contact.

17. Procédé selon la revendication 15 ou 16, dans lequel la couche électroconductrice (4) est dotée de moyens de connexion (6, 7, 8, 9) pour établir une communication électrique avec un dispositif ou un instrument approprié.

18. Procédé selon l'une quelconque des revendications 15 à 17, dans lequel la couche électroconductrice (4) est dotée d'une première extrémité d'un fil approprié pour conduire le courant électrique et l'autre extrémité du fil est appropriée pour établir une connexion électrique avec un instrument ou un dispositif, directement ou via un fil de connexion approprié.

19. Procédé selon la revendication 18, dans lequel l'extrémité du fil est fixée à la couche électroconductrice (14) par une soudure ou un rivet.

20. Procédé selon l'une quelconque des revendications 15 à 19, dans lequel la partie intermédiaire amovible (10) comprend deux couches empilées ou plus.

21. Procédé selon l'une quelconque des revendications 15 à 20, dans lequel la partie intermédiaire (10), lorsqu'elle est enlevée, exerce des forces sur au moins l'un de ladite couche électroconductrice (4) et dudit gel électroconducteur (12), forçant ladite couche électroconductrice (4) et ledit gel électroconducteur (12) ensemble.
